# EUROPÄISCHE PATENTSCHRIFT

(11) **EP 1 506 750 B1**
(45) Veröffentlichungstag und Bekanntmachung des Hinweises auf die Patenterteilung: **22.04.2009**
(21) Anmeldenummer: 04014381.0
(22) Anmeldetag: 18.06.2004
(51) Int. Cl.: A61F 2/36

(54) **Oberschenkelteil einer Hüftgelenkendoprothese**
Femoral part of a hip joint endoprosthesis
Partie fémorale d'une endoprothèse de l'articulation de la hanche

(30) Priorität: 01.07.2003 DE 10329673
(43) Veröffentlichungstag der Anmeldung: 16.02.2005
(73) Patentinhaber: Smith & Nephew Orthopaedics AG, 6343 Rotkreuz (CH)
(72) Erfinder: DORN, Ulrich, Prim. Univ. Doz. Dr. med., A-5020 Salzburg (AT); BRACK, René, CH-6330 Cham (CH)
(74) Vertreter: Popp, Eugen

(56) Entgegenhaltungen:
- EP-A- 0 093 869
- EP-A- 0 112 435
- EP-A- 0 378 044
- DE-A- 4 428 099
- DE-A- 19 547 638

## Beschreibung

Die Erfindung betrifft ein Oberschenkelteil einer Hüftgelenkendoprothese, mit einem blattartigen Schaft, dessen Querschnitt vom distalen Ende zum proximalen Ende hin ständig zunimmt, wobei im proximalen Schaftbereich stufenförmige Absätze ausgebildet sind, und wobei die stufenförmigen Absätze sich etwa in Schaftlängsrichtung erstreckend an wenigstens einer der beiden Blattseiten ausgebildet sind, und von lateral nach medial eine zunächst ansteigende und dann wieder abfallende "Treppe" definieren.

Aus der EP 0 112 435 A1 ist ein Oberschenkelteil einer Hüftgelenkendoprothese bekannt, bei dem eine stufenweise Zunahme des Querschnitts zwischen der medialen und der lateralen Schmalseite vorgesehen ist. Diese Stufung ist mittels ventraler und dorsaler Anformungen realisiert, die vom proximalen Ende bis zum distalen Ende des Oberschenkelteils reichen. Durch diese Ausbildung ist beabsichtigt, eine Vergrößerung der Kontaktfläche zwischen Schaft und Knochenzement zu erreichen, um die Stabilität der Prothesenverankerung zu verbessern. Es sollen dadurch Luxationen vermieden, die auf polymerisationsbedingter Volumenverkleinerung des Knochenzements beruhen. Jedoch ist mittels dieses bekannten, stützkragenfreien Oberschenkelteils keine allen Anforderungen genügende Einleitung der vertikalen Last von der Hüfte in den Femur möglich, da die am distalen Schaftende vorgesehenen auslaufenden Erhebungen der ventralen/dorsalen Anformungen nicht als mit entsprechend konformen Ausnehmungen im Femur korrespondierende Stufen ausgebildet sind.

Aus der DE 33 10 486 A1 ist ein Endoprothesenschaft mit einer Vielzahl von Stufungen bekannt, welche sich jeweils auf den gesamten Umfangsbereich erstrecken. Bei dieser Ausführungsform ist die Einleitung der vertikalen Last in den Femur auf relativ kleine Flächen und vornehmlich auf das distale Ende des Oberschenkelteils beschränkt, während große Flächen des proximalen Schaftendes nur eine geringe Funktion bei der Krafteinleitung übernehmen.

Weiter sei noch auf die DE 195 47 638 C2 verwiesen, die ein Oberschenkelteil einer Hüftgelenkendoprothese mit einem Schaft beschreibt, dessen Querschnitt vom distalen Schaftende zum proximalen Schaftende hin ständig zunimmt und im proximalen Schaftbereich in medial/lateraler Richtung oval gestreckt ist. Im proximalen Schaftbereich erfolgt zum proximalen Schaftende hin die Zunahme des Querschnitts in ventral/dorsaler Richtung in mehreren Stufen, deren medial/laterale Breite von der distalen Stufenkante zum proximalen Schaftende hin zunimmt. Damit soll die formschlüssige Einleitung der vertikalen Last in den Femurknochen mit ausreichend großen Stützflächen realisiert werden.

Die stufenförmigen Absätze sind jedoch relativ kompliziert, nämlich etwa trapezförmig gestaltet mit der Folge, dass der Knochenhohlraum entsprechend aufwendig vorbehandelt werden muß, um einen Passsitz des Schaftes innerhalb des Knochenhohlraumes sicherzustellen.

Schließlich zeigt die DE 195 47 683 A1 einen Oberschenkelteil einer Hüftgelenkendoprothese gemäß dem Oberbegriff von Anspruch 1, der einen Schaft aufweist, dessen Querschnitt vom distalen Schaftende zum proximalen Schaftende hin ständig zunimmt und im proximalen Schaftbereich in medial/lateraler Richtung oval gestreckt ist. Zur verbesserten Verankerung des Oberschenkelteils in einem natürlichen Femurknochen erfolgt die Zunahme des Schaftquerschnitts zum proximalen Schaftende hin in ventral/dorsaler Richtung in mehreren Stufen, deren medial/laterale Breite von der distalen Stufenkante zum proximalen Schaftende hin zunimmt. Nachteil einer solchen Ausführungsform ist jedoch die hinsichtlich der Knochenanatomie unvorteilhafte Form des Oberschenkelteils der Hüftgelenkendoprothese.

Der vorliegenden Erfindung liegt die Aufgabe zugrunde, ein Oberschenkelteil der eingangs genannten Art zu schaffen, an das der Knochenhohlraum des Femurs einfach anpassbar ist und das sowohl eine hohe Primärstabilität als auch Langzeitstabilität sicherstellt und außerdem eine großflächige Einleitung vertikaler Lasten in den Knochen ermöglicht.

Diese Aufgabe wird erfindungsgemäß durch die kennzeichnenden Merkmale des Anspruches 1 gelöst.

Ein wesentlicher Punkt der vorliegenden Erfindung besteht also darin, dass die stufenförmigen Absätze sich etwa in Schaftlängsrichtung erstreckend an wenigstens einer der beiden Blattseiten ausgebildet sind und von lateral nach medial eine zunächst ansteigende und dann wieder abfallende "Treppe" definieren. Diese konstruktive Formgebung bedarf eine Anpassung des Knochenhohlraums nur in Femurlängsrichtung. Es müssen also keine Quer- oder Schrägstufen innerhalb des Knochenhohlraums mittels einer entsprechend komplizierten Raspel ausgebildet werden. Dementsprechend lässt sich auch wesentlich leichter, und zwar auch für einen etwas ungeübteren Operateur ein formschlüssiger Halt der Prothese innerhalb des Knochenhohlraums erreichen, so dass eine hohe Primärstabilität sowie auch Langzeitstabilität gewährleistet sind. Besonders hervorzuheben ist dabei noch die Ausbildung von stufenförmigen Absätzen an der ventralen Blattseite des Schaftes, während die dorsale Blattseite eine ebene Fläche bildet, da dies besonders anatomiegerecht ist.

Vorzugsweise steigen die stufenförmigen Absätze jeweils aus der Blattebene heraus von distal nach proximal an, und zwar insbesondere kontinuierlich. Damit lässt sich eine großflächige Einleitung vertikaler Kräfte in den Knochen erzielen. Abhängig von der Steigung der stufenförmigen Absätze wird ein mehr oder weniger ausgeprägter "proximaler Pressfit" erreicht. Die erfindungsgemäße Prothese eignet sich besonders gut für ein zementloses Implantieren. Wie bereits erwähnt, ist die Ausbildung bzw. Anpassung des Knochenhohlraums an die stufenförmigen Absätze des erfindungsgemäßen Oberschenkelteils mit einer entsprechend ausgebildeten Raspel in einfacher Weise möglich. Die Arbeitsbewegung der Raspel erfolgt lediglich in axialer Richtung, ohne dass auf die Positionierung horizontaler oder schräger Stufen geachtet werden muß. Die Anpassung des Hohlraumes geschieht in gleicher einfacher Weise wie für einen Schaft, dessen Oberflächen glatt-eben ausgebildet sind.

Bei Bedarf ist es jedoch auch denkbar, dass die stufenförmigen Absätze von distal nach proximal stufenförmig ansteigen. Dann ist die Anpassung des Knochenhohlraums etwas aufwendiger. Dieser Aufwand muß jedoch dann in Kauf genommen werden, wenn die äußeren Gegebenheiten dies erfordern. Die einfachere Ausführungsform stellt jedoch diejenige mit kontinuierlich von distal nach proximal ansteigenden stufenförmigen Absätze dar.

Weitere konstruktive Details und vorteilhafte Ausführungen sind in den Unteransprüchen beschrieben.

Bezüglich der Anpassung des Knochenhohlraumes ist dabei die Ausführungsform mit stufenförmigen Absätzen hervorzuheben, deren Breite von distal nach proximal jeweils konstant ist.

Diese Ausführungsform erlaubt ein "Nachsetzen" des Schaftes bei Änderung des "pressfit's" aufgrund einer Änderung der Knochenstruktur, insbesondere bei älteren Patienten. Durch dieses "Nachsetzen" wird eine vorteilhafte Sekundärstabilität erreicht und einer Lockerung des Schaftes, insbesondere im distalen Bereich desselben, auch nach längerer Zeit der Implantation vermieden.

Nachstehend wird eine bevorzugte Ausführungsform eines erfindungsgemäß ausgebildeten Oberschenkelteils anhand der beigefügten Zeichnung näher erläutert. Diese zeigt in:
- Fig. 1: Die Ansicht eines erfindungsgemäß ausgebildeten Oberschenkelteils in Ansicht von ventral bzw. anterior;
- Fig. 2: das Oberschenkelteil gemäß Fig. 1 in Ansicht von dorsal bzw. posterior;
- Fig. 3: den Schaft gemäß den Fig. 1 und 2 in Ansicht von lateral; und
- Fig. 4: den Schaft gemäß den vorstehenden Figuren in Ansicht von proximal nach distal.

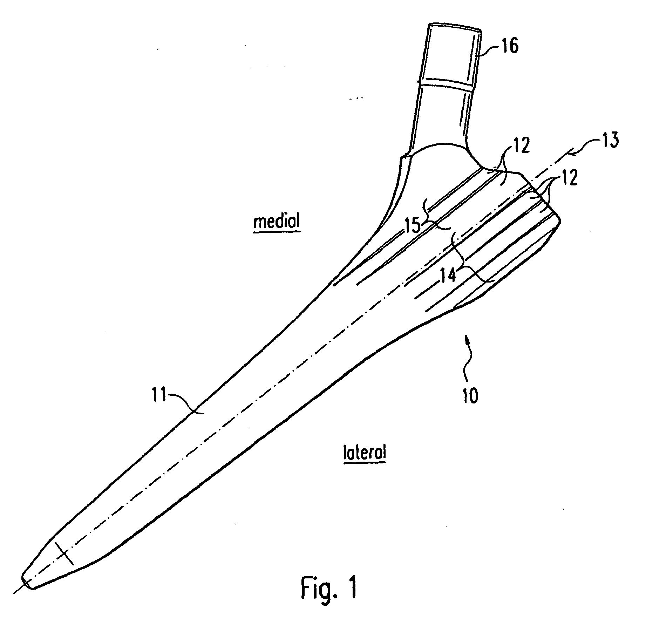

In den Fig. 1-4 ist ein Ausführungsbeispiel eines erfindungsgemäßen Oberschenkelteils 10 einer Hüftgelenkendoprothese dargestellt, welches einen blattartigen Schaft aufweist, dessen Querschnitt vom distalen Ende zum proximalen Ende hin ständig zunimmt (siehe Fig. 1 und 2 sowie Fig. 3). Im proximalen Schaftbereich sind an der ventralen Seite (Fig. 1) stufenförmige Absätze 12 ausgebildet, und zwar sich jeweils etwa in Schaftlängsrichtung, d.h. parallel zur Schaftlängsmittenachse 13 erstreckend. Diese stufenförmigen Absätze 12 definieren von lateral nach medial eine zunächst ansteigende und dann wieder abfallende "Treppe". Der von lateral nach medial ansteigende Treppenabschnitt ist mit der Bezugsziffer 14, und der von lateral nach medial abfallende Treppenabschnitt ist mit der Bezugsziffer 15 gekennzeichnet.

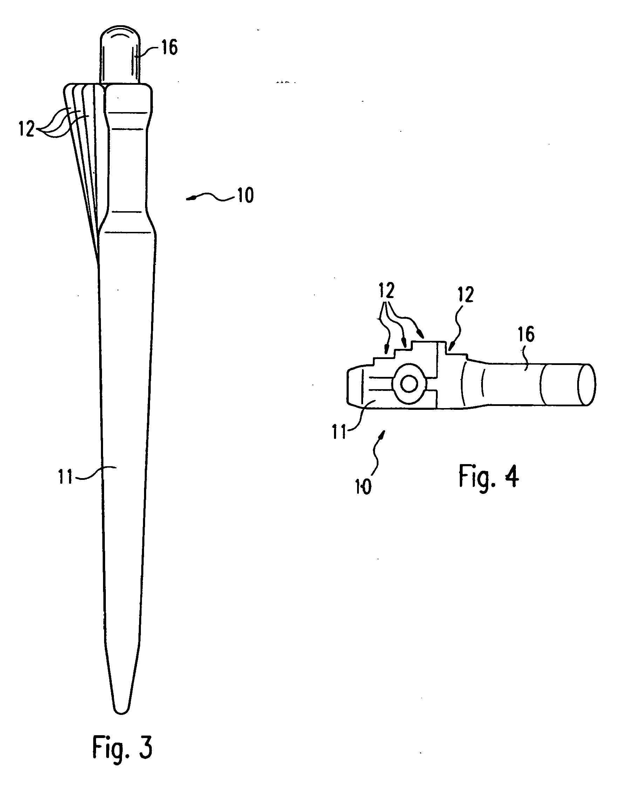

Des weiteren lassen die anliegenden Figuren, insbesondere auch Fig. 3 erkennen, dass die stufenförmigen Absätze 12 jeweils aus der Blattebene heraus von distal nach proximal ansteigen, und zwar jeweils linear. Denkbar ist auch ein leicht progressiver Anstieg entsprechend der Kontur eines Parabel- oder Hyperbelastes oder auch Ellipsen-Abschnitts.

Die Fig. 1 läßt erkennen, dass die durch die stufenförmigen Absätze 12 definierte Treppe von lateral bis zur Schaftlängsmittenachse 13, und zwar gemäß Fig. 1 sogar geringfügig darüber hinaus, ansteigt, um dann nach medial wieder abzufallen.

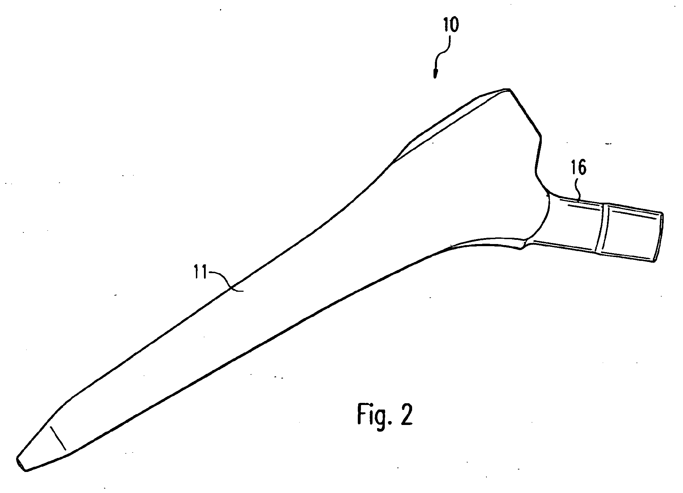

Entsprechend Fig. 2 ist die dorsale bzw. posteriore Seite des Schaftes 11 als ebene Fläche ausgebildet.

Bei der dargestellten Ausführungsform weist der von lateral nach medial ansteigende Treppenabschnitt 14 mehr, nämlich drei Stufen aus als der abfallende Treppenabschnitt 15, der bei der dargestellten Ausführungsform nur zwei Stufen umfaßt.

Entsprechend Fig. 1 weist des weiteren der höchste bzw. gegenüber der Blattebene erhabenste stufenförmige Absatz 12 die vergleichsweise größte Breite auf. Im übrigen ist die Breite der stufenförmigen Absätze 12 von distal nach proximal jeweils konstant. Die stufenförmigen Absätze 12 sind also von distal nach proximal streifenförmig ausgebildet.

Die Stufenhöhe der stufenförmigen Absätze 12 ist vorzugsweise jeweils gleich bemessen. Es kann jedoch vorteilhaft sein, die Stufenhöhe von lateral nach medial im Bereich des ansteigenden Treppenabschnitts 14 progressiv zunehmend und im Bereich des abfallenden Treppenabschnitts 15 degressiv abnehmend zu bemessen. Letztlich hängt dies von den anatomischen Gegebenheiten des Patienten ab. Im übrigen sind die stufenförmigen Absätze 12 sowohl an der lateralen als auch medialen Seite jeweils aus der Blattebene heraus ansteigend ausgebildet. Das heißt, die jeweils unterste Stufe erstreckt sich in der Blattebene, von der dann ausgehend die stufenförmigen Absätze in Richtung zur Schaftlängsmittenachse 13 hin ansteigen.

Entsprechend den Figuren 3 und 4 erstrecken sich die stufenförmigen Absätze etwa parallel zur Blattebene. Es ist jedoch auch denkbar, dass die Flachseiten der Absätze 12 zur Schaftlängsmittenachse 13 hin jeweils geringfügig ansteigend ausgebildet sind, und zwar entweder linear oder progressiv. Auch ist es zum Abbau von Spannungsspitzen denkbar, die Übergänge im Stufenbereich abgerundet auszubilden.

Zu dem in den Figuren 1-4 dargestellten Schaft sei noch erwähnt, dass am proximalen Schaftende ein Steckkonus 16 angeformt ist, der gegenüber der Schaftlängsmittenachse 13 um den sog. CCD-Winkel geneigt ausgerichtet ist. Der Steckkonus 16 dient dazu, einen Prothesenkopf (nicht dargestellt) mit entsprechender Konusausnehmung aufstecken und somit klemmend und fest am Oberschenkelteil befestigen zu können. Alternativ ist es auch möglich, am proximalen Ende des Schaftes einen Prothesenhals mit anschließendem Prothesenkopf einstückig anzuformen.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen

- 10: Oberschenkelteil
- 11: Schaft
- 12: stufenförmige Absätze
- 13: Schaftlängsmittenachse
- 14: lateralseitiger Treppenabschnitt
- 15: medialseitiger Treppenabschnitt
- 16: Steckkonus

## Patentansprüche

1. Oberschenkelteil (10) einer Hüftgelenkendoprothese, mit einem blattartigen Schaft (11), dessen Querschnitt vom distalen zum proximalen Ende hin ständig zunimmt, wobei im proximalen Schaftbereich stufenförmige Absätze (12) ausgebildet sind, und wobei
die stufenförmigen Absätze (12) sich etwa in Schaftlängsrichtung erstreckend an wenigstens einer der beiden Blattseiten ausgebildet sind, und von lateral nach medial eine zunächst ansteigende (14) und dann wieder abfallende "Treppe" (15) definieren,
**dadurch gekennzeichnet, dass**
die stufenförmigen Absätze (12) nur an der ventralen Blattseite des Schaftes (11) ausgebildet sind, während die dorsale Blattseite eine ebene Fläche bildet.

2. Oberschenkelteil nach Anspruch 1,
**dadurch gekennzeichnet, dass**
die stufenförmigen Absätze (12) jeweils aus der Blattebene heraus von distal nach proximal ansteigen.

3. Oberschenkelteil nach Anspruch 2,
**dadurch gekennzeichnet, dass**
die stufenförmigen Absätze (12) von distal nach proximal entweder stufenförmig oder kontinuierlich, und dabei jeweils entweder linear oder progressiv ansteigen.

4. Oberschenkelteil nach einem der Ansprüche 1 bis 3,
**dadurch gekennzeichnet, dass**
die durch die stufenförmigen Absätze (12) definierte "Treppe" von lateral bis zur Schaftlängsmittenachse (13) oder geringfügig darüber hinaus ansteigt, um dann nach medial wieder abzufallen.

5. Oberschenkelteil nach einem der Ansprüche 1 bis 4,
**dadurch gekennzeichnet, dass**
der von lateral nach medial ansteigende Treppenabschnitt (14) mehr, insbesondere drei Stufen aufweist, als der von lateral nach medial abfallende Treppenabschnitt (15).

6. Oberschenkelteil nach einem der Ansprüche 1 bis 5,
**dadurch gekennzeichnet, dass**
der höchste bzw. gegenüber der Blattebene erhabenste stufenförmige Absatz (12) die vergleichsweise größte Breite aufweist.

7. Oberschenkelteil nach einem der Ansprüche 1 bis 6,
**dadurch gekennzeichnet, dass**
die Stufenhöhe der stufenförmigen Absätze (12) jeweils gleich, oder alternativ von lateral nach medial im Bereich des ansteigenden Treppenabschnitts (14) progressiv zunehmend und im Bereich des abfallenden Treppenabschnitts (15) degressiv abnehmend bemessen ist.

8. Oberschenkelteil nach einem der Ansprüche 1 bis 7,
**dadurch gekennzeichnet, dass**
die stufenförmigen Absätze (12) lateral und medial jeweils aus der Blattebene heraus in Richtung zur Schaftlängsmittenachse (13) hin ansteigend ausgebildet sind.

9. Oberschenkelteil nach einem der Ansprüche 1 bis 8,
**dadurch gekennzeichnet, dass**
die Breite der stufenförmigen Absätze (12) von distal nach proximal jeweils konstant ist.

## Claims

1. Femoral portion (10) of a hip joint endoprosthesis, having a blade-like stem (11), the cross-section of which increases constantly from the distal end to the proximal end, wherein step-like shoulders (12) are formed in the proximal stem region, and wherein
the step-like shoulders (12) are formed on at least one of the two sides of the blade so as to extend approximately in the longitudinal direction of the stem and, from lateral to medial, define a "staircase" (15) which initially ascends (14) and then descends again,
**characterized in that**
the step-like shoulders (12) are formed only on the ventral side of the blade of the stem (11), while the dorsal side of the blade forms a planar surface.

2. Femoral portion according to claim 1,
**characterized in that**
the step-like shoulders (12) each rise out of the plane of the blade from distal to proximal.

3. Femoral portion according to claim 2,
**characterized in that**
the step-like shoulders (12) rise from distal to proximal either step-like or continuously, and in each case either linearly or progressively.

4. Femoral portion according to any one of claims 1 to 3,
**characterized in that**
the "staircase" defined by the step-like shoulders (12) ascends from the lateral as far as or slightly beyond the longitudinal centre axis (13) of the stem and then descends again towards the medial.

5. Femoral portion according to any one of claims 1 to 4,
**characterized in that**
the staircase portion (14) that ascends from lateral to medial has more steps, especially three steps, than the staircase portion (15) that descends from lateral to medial.

6. Femoral portion according to any one of claims 1 to 5,
**characterized in that**
the highest step-like shoulder (12), that is to say the shoulder most elevated with respect to the plane of the blade, has comparatively the greatest width.

7. Femoral portion according to any one of claims 1 to 6,
**characterized in that**
the step height of the step-like shoulders (12) is the same in each case, or alternatively is dimensioned from lateral to medial so as to increase progressively in the region of the ascending staircase portion (14) and to decrease degressively in the region of the descending staircase portion (15).

8. Femoral portion according to any one of claims 1 to 7,
**characterized in that**
the step-like shoulders (12) are formed so as to rise laterally and medially in each case out of the plane of the blade in the direction towards the longitudinal centre axis (13) of the stem.

9. Femoral portion according to any one of claims 1 to 8,
**characterized in that**
the width of the step-like shoulders (12) is in each case constant from distal to proximal.

## Revendications

1. Partie fémorale (10) d'une endoprothèse de la hanche, comprenant une tige de forme aplatie (11) dont la section transversale augmente constamment de l'extrémité distale à l'extrémité proximale, des décrochements en gradins (12) étant formés dans la région proximale de la tige et
lesdits décrochements en gradin (12) s'étendant sensiblement dans la direction longitudinale de la tige sur au moins l'une des deux faces de la forme aplatie et définissant, du latéral au médial, un « escalier » d'abord montant (14) puis redescendant (15),
**caractérisée en ce que** les décrochements en gradins (12) ne sont prévus que sur la face ventrale de la forme aplatie formant la tige (11), tandis que la face dorsale de ladite forme aplatie définit une surface plane.

2. Partie fémorale selon la revendication 1, **caractérisée en ce que** chacun des décrochements en gradins (12) ressort progressivement du plan de la forme aplatie du distal au proximal.

3. Partie fémorale selon la revendication 2, **caractérisée en ce que** les décrochements en gradins (12) montent du distal au proximal soit par paliers, soit en continu, et dans ce dernier cas de manière soit linéaire, soit progressive.

4. Partie fémorale selon l'une quelconque des revendications 1 à 3, **caractérisée en ce que** 1'« escalier » défini par les décrochements en gradins (12) monte du latéral jusqu'à l'axe médian longitudinal (13) de la tige voire légèrement au-delà, pour ensuite redescendre vers le médial.

5. Partie fémorale selon l'une quelconque des revendications 1 à 4, **caractérisée en ce que** le tronçon d'escalier (14) qui monte du latéral au médial compte plus de marches, en particulier trois, que le tronçon d'escalier (15) qui descend du latéral au médial.

6. Partie fémorale selon l'une quelconque des revendications 1 à 5, **caractérisée en ce que** le décrochement en gradins (12) le plus élevé ou le plus éloigné du plan de la forme aplatie a comparativement la plus grande largeur.

7. Partie fémorale selon l'une quelconque des revendications 1 à 6, **caractérisée en ce que** la hauteur de gradin des décrochements en gradins (12) est à chaque fois identique ou, en variante, du latéral au médial, en augmentation graduelle dans la région du tronçon d'escalier montant (14) et en diminution graduelle dans la région du tronçon d'escalier descendant (15).

8. Partie fémorale selon l'une quelconque des revendications 1 à 7, **caractérisée en ce que**, du côté latéral et du côté médial, les décrochements en gradins (12) sont conformés de manière à s'éloigner de plus en plus du plan de la forme aplatie en direction de l'axe médian longitudinal (13) de la tige.

9. Partie fémorale selon l'une quelconque des revendications 1 à 8, **caractérisée en ce que** la largeur des décrochements en gradins (12) est constante du distal au proximal.
